# EUROPEAN PATENT SPECIFICATION

(11) **EP 4 084 619 B1**
(45) Date of publication and mention of the grant of the patent: **04.09.2024**
(21) Application number: 20838928.8
(22) Date of filing: 15.12.2020
(51) Int. Cl.: C12R 1/46, A23C 9/127, A23C 9/123, A23C 9/13, C12N 1/20, C12R 1/225

(54) **NOVEL USE OF FORMATE**
NEUARTIGE VERWENDUNG VON FORMIAT
NOUVELLE UTILISATION DE FORMIATE

(30) Priority: 30.12.2019 NL 2024589
(43) Date of publication of application: 09.11.2022
(73) Proprietor: DSM IP Assets B.V., 6411 TE Heerlen (NL); CSK Food Enrichment BV, 8938 AS Leeuwarden (NL)
(72) Inventor: HAFKAMP, Albertus Antonius Gerardus, 6100 AA ECHT (NL); ZELDENRUST, Lisa, 6100 AA ECHT (NL); MEIJER, Willem Cornelis, 6100 AA ECHT (NL)
(74) Representative: dsm-firmenich IP
(86) International application number: PCT/EP2020/086258
(87) International publication number: WO 2021/136654

(56) References cited:
- WO-A1-2012/076665
- CN-A- 103 571 775
- US-A1- 2011 236 530
- US-B2- 10 368 559
- BEAL C ET AL: "Combined Effects of Culture Conditions and Storage Time on Acidification and Viscosity of Stirred Yogurt", JOURNAL OF DAIRY SCIENCE, AMERICAN DAIRY SCIENCE ASSOCIATION, US, vol. 82, no. 4, 1 April 1999 (1999-04-01), pages 673 - 681, XP027110994, ISSN: 0022-0302, [retrieved on 19990401]
- KURULTAY S ET AL: "Determination of the effects of different amino acids, Sodium Formate and their combinations on some growth characteristics of mixed and single cell cultures of yoghurt bacteria", JOURNAL OF TEKIRDAG AGRICULTURAL FACULTY, TRAKYA UNIVERSITESI, AGRICULTURAL FACULTY, vol. 2, no. 2, 1 January 2005 (2005-01-01), pages 153 - 160, XP002521305, ISSN: 1302-7050
- JUNKO NISHIMURA ET AL: "Effect of Formic Acid on Exopolysaccharide Production in Skim Milk Fermentation by Lactobacillus delbrueckii subsp. bulgaricus OLL1073R-1", BIOSCIENCE OF MICROBIOTA, FOOD AND HEALTH, vol. 32, no. 1, 1 January 2013 (2013-01-01), pages 23 - 32, XP055586893, DOI: 10.12938/bmfh.32.23

## Description

### Field

The invention is in the field of fermented milk-based compositions and relates to the use of formate in milk-based compositions and/or starter cultures.

### Background

Fermented milk-based based compositions include yoghurt and fresh cheese such as cottage cheese, cream cheese, quark and fromage blanc. Fermented milk-based based compositions are appreciated for their textural properties such as thickness. Thickness may to a large extent be derived from the protein present in the composition. For this aspect, it is assumed that in general there is a positive correlation between the amount of protein, thickness and the viscosity. CN103571775 describes an exocellular polysaccharide producing Streptococcus thermophilus bacterial strain for improving fermented milk viscosity.

At the same time, manufacturers of fermented milk-based compositions seek to exploit their production installations as efficiently as possible. A higher acidification rate contributes to this as it shortens fermentation time and therefore increases the production capacity.

However, a higher acidification rate is known to have a negative influence on the thickness of the resulting fermented milk-based composition. Beal et al., in their article titled "Combined Effects of Culture Conditions and Storage Time on Acidification and Viscosity of Stirred Yogurt", published in J. Dairy Science, Vol. 82(4), p. 673, 1999, indicated that the factors affecting yogurt acidity and viscosity had never been studied together, and aimed to display the combined effect of four main working conditions (strain association, incubation temperature, final fermentation pH, and storage time) by using an experimental design on the bacterial concentrations, the acidification activity, the postacidification, and the viscosity of stirred yogurt. In their conclusions they found there was a relationship between acidification and texture, namely that high viscosity was related to slow acidification (i.e., long fermentation time).

When additional formate is added to a milk-based composition that is to be fermented in the presence of lactic acid bacteria, formate is known to increase the acidification rate of said bacteria in many cases. For example in WO 2012/076665 A1 a method is described to increase the acidification rate beyond the known acceleration of the acidification rate by formate by combining the formate with separately cultured frozen microorganisms in a starter culture.

Kurultay et al, in their article titled "Determination of The Effects of Different Amino Acids, Sodium Formate and Their Combinations on Some Growth Characteristics of Mixed and Single Cell Cultures of Yoghurt Bacteria", published in the Journal of Tekirdag Agricultural Faculty, Kurultay ve ark. Vol. (2)2, p. 153, 2005, aimed to determine the stimulatory or inhibitory effects of the different amino acids, sodium formate and combination of these compounds on the metabolic activity of mixed and single cell cultures of yoghurt bacteria. In their laboratory experiments, before inoculation, predetermined amounts sodium formate (Sf) (500 ppm) were added into the milk. Sodium formate was stated to show a clear effect on the titratable acidity contents of the samples.

In view of the combined teachings of Beal et al and WO 2012/076665 or Kurultay et al. one skilled in the art would expect that the addition of formate would have a negative influence on the thickness and viscosity of the resulting fermented milk-based composition.

Nishimura et al., in their article titled "Effect of Formic Acid on Exopolysaccharide Production in Skim Milk Fermentation by Lactobacillus delbreuckii subsp. bulgaricus OLL1073R-1", published in Bioscience of Microbiota, Food and Health Vol. 32 (1), p. 23, 2013, indicate that in yogurt fermentation, *L. bulgaricus* utilizes formic acid supplied from S. *thermophilus.* Nishimura et al. subsequently investigated the effect of formate on the EPS production in skim milk by *L. bulgaricus* OLL1073R-1. In their laboratory experiments skim milk (10%) with or without formate was prepared at a concentration of 100 mg/l by addition sodium formate (10 mg/100 mL, 1.47 mM) based on formic acid production from S. *thermophilus* of 40 to 600 mg/l. The media were subsequently sterilized by low-temperature longtime pasteurization to avoid the formation of formic acid. The *L. bulgaricus* culture was subsequently inoculated into 10% skim milk with or without formate and incubated at 37°C for 24 hr. No effect on viscosity was established.

At the end of their discussion section, Nishimura et al. state that the control of formic acid levels is important to the regulation of EPS production of *L. bulgaricus* OLL1073R-1 in yogurt fermentation. Nishimura et al refer to data stated to show that the increase of EPS production is closely related to cell numbers, indicating that EPS production is dependent on cell proliferation rate. However, Nishimura et al do not teach any direct effect of formic acid levels on EPS production. Further, Nishimura et al do not provide any teaching on the type of EPS or the length of the EPS involved. Also Nishimura et al., do not suggest adding formic acid (as such) to industrial fermentations, but merely suggest that the selection and application of strains of S. *thermophilus* with high formic acid production would be useful for the development of EPS-rich yogurt cultures in the future.

Consumers expect a certain thickness of their fermented milk-based composition which - in the absence of additives or texturizing strains - would require a certain protein level in the fermented milk-based composition. As protein is a costly component in a fermented milk-based composition, manufacturers are constantly searching for ways to lower the protein level whilst keeping product parameters such as thickness constant. In addition, manufacturers of fermented milk-based compositions seek to exploit their production installations as efficiently as possible. As indicated above, a higher acidification rate contributes to this as it shortens fermentation time and therefore increases the production capacity.

It would be an advancement in the art to provide a method to (further) improve thickness of a fermented milk-based composition.

### Summary

The inventors now surprisingly found that by adding formate to a milk-based composition before and/or during fermenting the composition, the fermented milk-based composition demonstrates a higher viscosity and a higher thickness, i.e. a higher sensory mouthfeel attribute thickness, as compared to the fermented milk-based composition without adding formate before fermenting.

Accordingly, the invention provides a new use of formate for increasing the thickness of a fermented milk-based composition, wherein the fermented milk-based composition is fermented by S. *thermophilus* and the formate is provided to the milk-based composition before and/or during fermentation, preferably before fermentation.

In addition, the invention provides a new use of formate in a starter culture for increasing the thickness of a fermented milk-based composition, wherein the starter culture comprises S. *thermophilus.* The invention is defined by the appended set of claims.

Example 1 demonstrates this effect for a milk-based composition fermented by S. *thermophilus;* example 2 demonstrates this effect for a milk-based composition fermented by a combination of S. *thermophilus* and *L. bulgaricus.*

The invention therefore provides manufacturers of fermented dairy compositions the opportunity to offer products with either increased thickness for an equal protein level, or products with a lower protein level with a similar thickness.

Such effect has not been disclosed before and is unexpected considering that formate is known to increase the fermentation rate and an increased fermentation rate is known to lower the thickness of the resulting fermented milk-based composition. Moreover, although formate is known to stimulate the formation of EPS in a specific *L. bulgaricus* strain when *L. bulgaricus* is the single fermenting bacteria, no effect on thickness has been observed. Considering the interaction between S. *thermophilus* and *L. bulgaricus* present when fermenting a milk-based composition, the learnings of tests with exclusively *L. bulgaricus* anyhow cannot provide direction for a mixed culture of S. *thermophilus* and *L. bulgaricus.*

For cultures comprising S. *thermophilus* as a single strain no effect on thickness is expected as S. *thermophilus* does not metabolize formate while fermenting milk-based compositions; instead its metabolism produces formate.

An additional benefit of the current invention is that in addition to the effect on thickness the acidification rate is increased. Such increased acidification rate shortens the fermentations time. A shorter fermentation time may lower the production costs. The invention therefore also relates to the combined decrease of the fermentation time and increase of the thickness of the fermented milk-based composition.

### Detailed description

In this document and in its claims, the verb "to comprise" and its conjugations are used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

Unless explicitly indicated otherwise, the embodiments and preferences as outlined below can be combined in any desired manner to carry out the above invention.

In one aspect, the invention thus relates to the use of formate for increasing the thickness of a fermented milk-based composition, wherein the fermented milk-based composition is fermented by S. *thermophilus* and the formate is provided to the milk-based composition before and/or during fermentation, preferably before fermentation, wherein increasing the thickness is selected from the group consisting of increasing the viscosity, increasing the sensory mouthfeel attribute thickness and the combination thereof. The new use of formate also includes the use of formate for increasing of the viscosity of a fermented milk-based composition, wherein the fermented milk-based composition is fermented by S. *thermophilus* and the formate is provided to the milk-based composition before and/or during fermentation, preferably before fermentation. As indicated above, the increase of thickness, respectively viscosity, can be determined vis-à-vis the situation where no formate was added before and/or during fermentation.

In another aspect, the invention also relates to the use of formate in a starter culture, for increasing the thickness of a fermented milk-based composition, wherein the starter culture comprises *S*. *thermophilus,* wherein increasing the thickness is selected from the group consisting of increasing the viscosity, increasing the sensory mouthfeel attribute thickness and the combination thereof. The new use of formate also includes the use of formate in a starter culture, for increasing the viscosity of a milk-based composition fermented with said starter culture, wherein said starter culture comprises S. *thermophilus..* As indicated above, the increase of thickness, respectively viscosity, can be determined vis-à-vis the a situation where no formate was added before and/or during fermentation.

### Increasing the thickness

The invention concerns the use of formate for increasing the thickness of a fermented milk-based composition. The thickness of a fermented milk-based composition can be established through rheological measurements and/or through sensory measurements.

Rheological parameters that can be determined by rheological measurements and that are positively correlated with thickness are the shear storage modulus, the shear loss modulus and the viscosity. In the context of the invention, the viscosity referred to can be measured in a rheometer using a cone-plate geometry at 20 °C at a shear rate of 100 s⁻¹. In the context of the invention, the shear storage modulus and/or the shear loss modulus referred to can be measured in a rheometer using a cone-plate geometry at 20 °C. The rheometer can be operated in rotational mode or oscillatory mode.

Sensory measurements can be performed by an expert panel. In an expert panel the panelist are trained to or experienced in objectively assessing sensory parameters. Sensory parameters are also referred to as sensory attributes. Sensory attributes can be visual attributes, mouthfeel attributes, taste attributes and smell attributes. Typical mouthfeel attributes in the context of fermented milk-based compositions are thickness, sliminess, graininess and roughness. The sensory attribute mouthfeel thickness is the thickness as perceived in the mouth.

Sensory measurements and/or viscosity measurements are typically used in the production of fermented milk-based compositions as a quality monitoring tool for thickness.

The invention concerns the use of formate for increasing the thickness of a fermented milk-based composition, wherein increasing the thickness is selected from the group consisting of increasing the viscosity, increasing the sensory mouthfeel attribute thickness and a combination therefore. Even more preferably, the invention concerns the use of formate for increasing the thickness of a fermented milk-based composition, wherein increasing the thickness is selected from the group consisting of increasing the viscosity and increasing the sensory mouthfeel attribute thickness. Most preferably the invention concerns the use of formate for increasing the thickness of a fermented milk-based composition, wherein increasing the thickness is increasing the viscosity.

Preferably the viscosity is measured in a rheometer using a cone-plate geometry at 20 °C at a shear rate of 100 s⁻¹. Preferably the sensory attribute mouthfeel thickness is established by an expert panel.

The present invention thus relates to the use of formate for increasing the thickness of a fermented milk-based composition, wherein the fermented milk-based composition is fermented by S. *thermophilus,* wherein the formate is provided to the milk-based composition before and/or during fermentation, preferably before fermentation and wherein increasing the thickness is selected from the group consisting of increasing the viscosity, increasing the sensory mouthfeel attribute thickness and the combination thereof.

In a preferred embodiment the invention relates to the use of formate for increasing the viscosity of a fermented milk-based composition, wherein the fermented milk-based composition is fermented by S. *thermophilus,* wherein the formate is provided to the milk-based composition before and/or during fermentation, preferably before fermentation. In another preferred embodiment the invention relates to the use of formate for increasing the sensory mouthfeel attribute thickness of a fermented milk-based composition, wherein the fermented milk-based composition is fermented by S. *thermophilus,* wherein the formate is provided to the milk-based composition before and/or during fermentation, preferably before fermentation. In yet another preferred embodiment the invention relates to the use of formate for increasing the viscosity and increasing the sensory mouthfeel attribute thickness of a fermented milk-based composition, wherein the fermented milk-based composition is fermented by S. *thermophilus,* wherein the formate is provided to the milk-based composition before and/or during fermentation, preferably before fermentation.

The present invention further relates to the use of formate in a starter culture for increasing the thickness of a fermented milk-based composition, wherein the starter culture comprises S. *thermophilus,* and wherein increasing the thickness is selected from the group consisting of increasing the viscosity, increasing the sensory mouthfeel attribute thickness and the combination thereof.

Preferably the use of formate is a use wherein the thickness, the viscosity and/or the sensory mouthfeel attribute thickness is/are increased as compared to a reference fermented milk-based composition fermented by S. *thermophilus* wherein no formate is provided to the reference milk-based composition before and during fermentation or as compared to a starter culture not comprising formate.

In a preferred embodiment the invention relates to the use of formate in a starter culture for increasing the viscosity of a fermented milk-based composition, wherein the starter culture comprises S. *thermophilus.* In another preferred embodiment the invention relates to the use of formate in a starter culture for increasing the sensory mouthfeel attribute thickness of a fermented milk-based composition, wherein the starter culture comprises S. *thermophilus.* In another preferred embodiment the invention relates to the use of formate in a starter culture for increasing the viscosity and increasing the sensory mouthfeel attribute thickness of a fermented milk-based composition, wherein the starter culture comprises S. *thermophilus.*

### Formate

The invention concerns the use of formate. Formate is the anion derived from formic acid, characterized by the formula CHOO⁻. Herein, when reference is made to the weight of formate, the weight of the formate anion is meant. The "use of formate" is herein understood to include the use of formic acid, a formate salt and/or a formate ester thereof. Preferably formate is provided or used as formic acid (CHOOH), a formate salt and/or a formate ester. Preferably for the current invention formate is selected from the group consisting of formic acid, sodium formate, potassium formate, ammonium formate, calcium formate, magnesium formate, ferric formate, zinc formate, copper formate, ethyl formate, methyl formate and any combination thereof. More preferably, formate is selected from the group consisting of formic acid, sodium formate, potassium formate, ammonium formate, calcium formate and any combination thereof. Yet more preferably, the formate is provided in the form of formic acid, an alkaline salt of formic acid, an alkaline earth salt of formic acid or a combination of any of those. Even more preferably formate is selected from the group consisting of sodium formate, formic acid and a combination thereof. Most preferably formate is selected from the group consisting of sodium formate and formic acid.

### Fermented milk-based composition

The invention concerns the use of formate in a fermented milk-based composition, in order to increase the thickness of the fermented milk-based composition. A milk-based composition is herein understood to be a composition comprising milk components. The milk used as a basis for the fermented milk-based composition can suitably comprise milk from an animal or mammal source, and/or milk from a plant-based source. Preferably the milk used as a basis for the fermented milk-based composition is from an animal or mammal source. Milk components are known in the art and comprise milk components originating from animal milk such as milk protein, lactose and milk fat. Alternatively or additionally, in the case of plant milk such as almond milk, oat milk and soy milk, milk components may be from non-animal origin comprising a combination of one or more plant proteins, one or more sugar sources and optionally one or more sources of fat. The sugar source can be utilized by the strain of S. *thermophilus;* preferably the plant proteins are selected from the group consisting of soy protein, oat protein and almond protein.

The milk is preferably selected from the group consisting of cow's milk, goats milk, sheep milk, almond milk, oat milk, and soy milk. In a preferred embodiment the milk is cow's milk. In an alternative preferred embodiment the milk is free of an animal's milk and comprises soy milk, oat milk or almond milk.

Preferably the milk-based composition comprises at least 50 wt.% milk components, more preferably at least 75 wt.% milk components, even more preferably at least 95 wt.% milk components, most preferably 100 wt.% milk components, wherein the weight percentage is based on dry weight of the milk components and on dry weight of the milk-based composition.

Preferably the milk components are selected from the group consisting of milk protein, lactose, milk fat, plant protein, sugar and plant fat. More preferably the milk components are selected from the group consisting of milk protein, lactose, milk fat; or alternatively more preferably the milk components are selected from the group consisting of plant protein, sugar and plant fat.

Preferably the milk-based composition comprises at least 50 wt.% milk components, more preferably at least 75 wt.% milk components, even more preferably at least 95 wt.% milk components, most preferably 100 wt.% milk components, wherein the weight percentage is based on dry weight of the milk components and on dry weight of the milk-based composition, wherein the milk components are selected from the group consisting of milk protein, lactose, milk fat, plant protein, sugar and plant fat.

It is further preferred that the milk-based composition comprises at least 50 wt.% milk components, more preferably at least 75 wt.% milk components, even more preferably at least 95 wt.% milk components, most preferably 100 wt.% milk components, wherein the weight percentage is based on dry weight of the milk components and on dry weight of the milk-based composition, wherein the milk components are selected from the group consisting of milk protein, lactose and milk fat.

The milk-based composition can suitably be fermented. In the context of milk-based compositions, fermentation is understood to be the process in which carbohydrates in the milk-based composition are converted to organic acids by bacteria under anaerobic conditions. A fermented milk-based composition is understood to be a milk-based composition that underwent fermentation. Herein, when an amount of milk components in a fermented milk-based composition is mentioned, the amount of milk components of the fermented milk-based composition before fermentation is taken as the amount of milk components in the fermented milk-based composition.

Preferably the fermented milk-based composition is selected from the group consisting of ripened cheese, fresh cheese and yoghurt, more preferably from the group consisting of fresh cheese and yoghurt. Most preferably the fermented milk-based composition has a spoonable consistency, such as the consistency of yoghurt. Most preferably the fermented milk-based product is a yoghurt.

That is, the use of formate is preferably a use wherein the fermented milk-based composition is selected from the group consisting of yoghurt and fresh cheese, preferably the fermented milk-based composition is yoghurt.

Preferably the yoghurt is a stirred-type yoghurt. A stirred type yoghurt is known in the art and concerns a yoghurt that is subjected to a shear treatment between fermentation and filling in a final package. In contrast, a set-type yoghurt is a yoghurt that is fermented in its final package. No further shear treatment is provided.

Yoghurt and cheese are both fermented milk-based compositions. The difference between yoghurt and cheese is that in the case of cheese whey is separated from the fermented product during or after fermentation. The difference between fresh cheese and ripened cheese is that ripened cheese is ripened whilst fresh cheese is not. For most cheeses rennet is added to the milk-based composition before fermentation, although for some fresh cheeses this is not the case.

Preferred fermented milk-based compositions are yoghurt and fresh cheese such as cottage cheese, cream cheese, quark and fromage blanc. These fermented milk-based based compositions are appreciated for their textural properties such as thickness and suitably may derive such thickness to a large extent from the protein present in the composition. Without wishing to be bound by any kind of theory, it is believed that in general there is a positive correlation between the amount of protein and the viscosity. Fermented dairy compositions are generally thicker than their non-fermented counter-parts already due to the coagulation of the milk proteins upon fermentation. Moreover in some cases upon fermentation polysaccharides are formed which may in some cases further contribute to the thickness of the fermented milk-based composition.

Hence, preferably the fermented milk-based composition comprises a protein. Preferably the fermented milk-based composition comprises in the range of 2 to 15 wt.% of protein based on total weight of the fermented milk-based composition, more preferably in the range of 2 to 10 wt.% of protein based on total weight of the fermented milk-based composition, even more preferably in the range of 3 to 8.5 wt.% of protein based on total weight of the fermented milk-based composition, and most preferably in the range of 3 to 5.5 wt.% of protein based on total weight of the fermented milk-based composition. Preferably the protein is a milk protein. In another preferred embodiment the protein comprises a plant-based protein; in yet another preferred embodiment the fermented milk-based composition comprises a protein wherein all proteins are plant-based proteins.

Preferably the fermented milk-based composition comprises sugar. Preferably the fermented milk-based composition comprises in the range of 2 to 10 wt.% of sugar based on total weight of the fermented milk-based composition, more preferably in the range of 3 to 8.5 wt.% of sugar based on total weight of the fermented milk-based composition, most preferably in the range of 3 to 5.5 wt.% of sugar based on total weight of the fermented milk-based composition. Preferably the fermented milk-based composition comprises lactose. Preferably the fermented milk-based composition comprises in the range of 2 to 10 wt.% of lactose based on total weight of the fermented milk-based composition, more preferably in the range of 3 to 8.5 wt.% of lactose based on total weight of the fermented milk-based composition, most preferably in the range of 3 to 5.5 wt.% of lactose based on total weight of the fermented milk-based composition.

As the milk comprises a sugar source which is partially converted to an acid upon fermentation, the fermented milk-based composition preferably comprises an amount of lactate and optionally an amount of a sugar. Lactate may be present as lactic acid and/or as lactate salt. Preferably the fermented milk-based composition comprises in the range of 2 to 20 wt.% of the sum of sugars and lactate based on total weight of the fermented milk-based composition.

Preferably the fermented milk-based composition comprises a fat. Preferably the fermented milk-based composition comprises in the range of 0 to 10 wt.% of fat based on total weight of the fermented milk-based composition, more preferably in the range of 0.1 to 8.5 wt.% of fat based on total weight of the fermented milk-based composition, most preferably in the range of 0.1 to 5 wt.% of fat based on total weight of the fermented milk-based composition. Preferably the fat comprises milk fat.

### Starter culture

In one aspect, the invention concerns the use of formate in a starter culture, in order to increase the thickness of a fermented milk-based composition upon fermentation by said starter culture. A starter culture is herein defined as a preparation containing bacterial cells that is intended for inoculating a medium to be fermented. Herein, the medium to be fermented is suitably a milk-based composition as defined above. The bacterial cells are the bacteria used in the fermentation. A 'bacterium' is a different wording for a 'bacterial cell', 'bacteria' is the plural of bacterium and is a different wording for 'bacterial cells'.

Bacteria that are able to convert lactose to lactate are known in the art as lactic acid bacteria (LAB). Examples of lactic acid bacteria genera are *Lactobacillus, Leuconostoc, Lactococcus* and *Streptococcus. S. thermophilus* is an abbreviation of *Streptococcus thermophilus, L. bulgaricus* is an abbreviation of *Lactobacillus delbrueckii subsp. bulgaricus.* Also *Lactobacillus delbrueckii subsp. bulgaricus* is a lactic acid bacterium.

In one embodiment, the invention relates to the use of formate in a starter culture, for increasing the thickness of a fermented milk-based composition, wherein the starter culture comprises S. *thermophilus.*

Preferably the starter culture comprises in the range of 0.1 to 90 wt.% formate based on total weight of the starter culture, more preferably in the range of 1 to 50 wt.% formate based on total weight of the starter culture, even more preferably in the range of 2 to 25 wt.% formate based on total weight of the starter culture, and most preferably in the range of 3 to 10 wt.% formate based on total weight of the starter culture.

The starter culture of the current invention suitably comprises S. *thermophilus.* More suitably, the starter culture may comprise at least one formic acid and/or formate producing S. *thermophilus* strain.

The starter culture may comprise, one, two or more S. *thermophilus* strains. Such S. *thermophilus* strains may include exopolysaccharide-producing S. *thermophilus* strains and/or S. *thermophilus* strains that do not produce exopolysaccharides (EPS). Preferably at least one of the S. *thermophilus* strains is a S. *thermophilus strain* that is capable of producing exopolysaccharides (EPS).

In a preferred embodiment the starter culture comprises a combination of S. *thermophilus* and *L. bulgaricus.* Also in these embodiments it is preferred that the starter culture comprises formate in the range of 0.1 to 90 wt.%, more preferably in the range of 1 to 50 wt.%, even more preferably in the range of 2 to 25 wt.% and most preferably in the range of 3 to 10 wt.%, all based on total weight of the starter culture. Without wishing to be bound by any kind of theory, it is believed that when yoghurt is produced by a combination of S. *thermophilus* and *L. bulgaricus* these bacteria have a specific interaction; S. *thermophilus* can provide formic acid, pyruvic acid, folic acid and carbon dioxide to *L. bulgaricus,* and then *L. bulgaricus* can promote growth of S. *thermophilus* by peptides or amino acids production, induced by the breakdown of proteins by *L. bulgaricus.* Both species may convert lactose to lactate.

In another preferred embodiment the starter culture exclusively comprises S. *thermophilus* as a source of lactic acid bacteria, in other words, in this embodiment the starter culture only comprises S. *thermophilus* as a source of lactic acid bacteria. 'Exclusively comprises' or 'only comprises' herein has the meaning that no other bacteria that are able to convert lactose to lactate are present in the starter culture. Hence, in one embodiment the starter culture is preferably a starter culture comprising lactic acid bacteria wherein such lactic acid bacteria consist of lactic acid bacteria of the genus *Streptococcus thermophilus.* In this embodiment it is preferred that the starter culture comprises S. *thermophilus* and that the starter culture comprises no other lactic acid bacteria. Also in this embodiment it is preferred that the starter culture comprises formate in the range of 0.1 to 90 wt.%, more preferably in the range of 1 to 50 wt.%, even more preferably in the range of 2 to 25 wt.% and most preferably in the range of 3 to 10 wt.%, all based on total weight of the starter culture.

Preferably the starter culture comprises at least 10⁹ CFU bacteria per gram of starter culture, more preferably at least 5·10⁹ CFU bacteria per gram of starter culture, even more preferably in the range of 5·10⁹ to 10¹² CFU bacteria per gram of starter culture, and most preferably in the range of 5·10⁹ to 10¹¹ CFU bacteria per gram of starter culture. CFU stands for colony forming units, which is known in the art. Also methods to determine the CFU per gram are known in the art. In the art a starter culture is commonly given an amount of CFU per gram or is referred to as comprising a specific CFU per gram. This amount refers to the amount of bacteria in the starter culture.

The starter culture may comprise other components in addition to bacterial cells.

It is preferred that the starter culture further comprises a cryoprotectant. A cryoprotectant is defined herein as a substance that protects the bacterial cells from damage during freezing and thawing of the starter culture. The cryoprotectant may be any additive, as long as it protects cells or tissues from damage during freezing and thawing. Cryoprotectants are known in the art. Preferably the cryoprotectant is selected from the group consisting of sugars, polyalcohols, polysaccharides, polyethers, antioxidants, oils, nucleosides, nucleotides and surfactants. More preferably the cryoprotectant is selected from the group consisting of a nucleoside, a nucleotide and a sugar. Most preferably the cryoprotectant is selected from the group consisting of lactose, sucrose, trehalose, inosine and an inosine derivative which is preferably inosine monophosphate. A cryoprotectant may be required for some bacterial genus and/or species. For other bacterial species or genus a cryoprotectant may not be required. If present, the starter culture preferably comprises in the range of 1 to 50 wt.% cryoprotectant based on total weight of the starter culture, more preferably in the range of 3 to 25 wt.% cryoprotectant based on total weight of the starter culture, most preferably in the range of 5 to 10 wt.% cryoprotectant based on total weight of the starter culture.

Preferably for the current invention the starter culture comprises lactose. Preferably the starter culture comprises in the range of 1 to 50 wt.% lactose per total weight of the starter culture, more preferably in the range of 3 to 25 wt.% lactose per total weight of the starter culture, most preferably in the range of 5 to 12 wt.% lactose per total weight of the starter culture.

In an embodiment of the invention the starter culture is a frozen or freeze-dried starter culture. Frozen and freeze-dried starter cultures are known in the art. Preferably the starter culture is a homogenous frozen or freeze-dried starter culture. A homogenous frozen or freeze-dried starter culture is a starter culture to which no components in an amount of more than 5 wt.%, preferably of more than 2 wt.% most preferably of more than 0 wt.% of the total weight of the starter culture are added after freezing or freeze drying. In another preferred embodiment the starter culture is a heterogeneous starter culture. A heterogeneous frozen or freeze-dried starter culture is a starter culture to which one or more components are added in a total amount of more than 5 wt.%, preferably of more than 2 wt.% most preferably of more than 0 wt.% of the total weight of the starter culture after freezing or freeze drying. Preferably the starter culture is a heterogeneous starter culture to which formate is added after freezing or freeze drying, more preferably the starter culture is a heterogeneous starter culture to which formate is added in the range of 2 to 25 wt.% formate based on total weight of the starter culture after freezing or freeze drying, most preferably in the range of 3 to 10 wt.% formate based on total weight of the starter culture after freezing or freeze drying.

### Fermentation of a milk-based composition

The process of fermentation is known in the art. The skilled person knows how to select the fermentation parameters for a specific fermentation process, based on the milk-based composition to be fermented, the targeted product and the starter culture used.

In one aspect of the present invention, formate is suitably provided to a milk-based composition before and/or during fermentation, preferably before fermentation of said milk-based composition. In this aspect, the invention thus suitably relates to the use of formate for increasing the thickness of a fermented milk-based composition, wherein the fermented milk-based composition has been fermented by S. *thermophilus* and wherein the formate is provided to the milk-based composition before and/or during fermentation, preferably before fermentation. Hence, for this aspect, the formate can suitably be provided to the milk-based composition as an additive separately from the starter culture, respectively separately from the S. *thermophilus.* For this aspect, the formate can suitably be added to the milk-based composition before addition of a starter culture, respectively S. *thermophilus;* and/or during fermentation but after addition of the starter culture, respectively S. *thermophilus.* If so desired, the formate can also be added at the exact same moment that the fermentation is started. That is, the formate can also be so used, wherein the starter culture, respectively S. *thermophilus,* is provided to the milk-based composition at the same moment as the formate is provided. A combination of several moments for addition of the formate is also possible.

The formate can thus be provided in a batch-wise, continuous, semi-continuous or fed-batch manner, before and/or during the fermentation.

In another aspect, the present invention relates to the use of formate in a starter culture for increasing the thickness of a fermented milk-based composition, wherein the starter culture comprises S. *thermophilus.* In these embodiments of the invention, formate is comprised in a starter culture when provided to the milk-based composition before fermentation. Preferred embodiments of the starter culture are as defined above. For this aspect the starter culture, respectively the S. *thermophilus,* can suitably be provided to the milk-based composition at the same moment as the formate is provided.

In other embodiments of the invention, formate is not comprised in a starter culture when provided to the milk-based composition before fermentation.

Preferably formate is provided in an amount of 1 to 500 microgram per gram of milk-based composition, more preferably formate is provided in an amount of 2 to 100 microgram per gram of milk-based composition, even more preferably formate is provided in an amount of 3 to 50 microgram per gram of milk-based composition, most preferably formate is provided in an amount of 3 to 15 microgram per gram of milk-based composition. The milk-based composition is the to be fermented milk-based composition. Preferably the formate is provided to the milk-based composition before and/or during fermentation, more preferably before fermentation.

The milk-based composition is fermented by *S. thermophilus.* With fermented by *S. thermophilus* is meant fermented by at least *S. thermophilus.* Other bacteria that ferment may also be present so other lactic acid bacteria may also be present or the milk based composition may be fermented by other lactic acid bacteria next to *S. thermophilus.*

In a preferred embodiment the milk-based composition is fermented by a combination of *S. thermophilus* and *L. bulgaricus.* More preferably the use of formate, for increasing the thickness of a fermented milk-based composition, is:
- a use of formate in a starter culture, wherein a milk-based composition is fermented by a combination of S. *thermophilus* and *L. bulgaricus;* or
- a use wherein a milk-based composition is fermented by a combination of *S. thermophilus* and *L. bulgaricus* and the formate is provided to the milk-based composition before and/or during fermentation, preferably before fermentation.

In another embodiment preferably the milk-based composition is exclusively fermented by *S. thermophilus* or in other words the milk-based composition is only fermented by *S. thermophilus.* In this case, the resulting fermented milk-based composition may suitably be a fermented milk-based composition that has been fermented exclusively by *S. thermophilus.* 'Exclusively fermented' or 'only fermented' has the meaning that no other bacteria that are able to convert lactose to lactate are provided to the to be fermented milk-based composition. Such bacteria are known in the art as lactic acid bacteria (LAB). Examples of lactic acid bacteria genera are *Lactobacillus, Leuconostoc, Lactococcus* and *Streptococcus. Lactobacillus delbrueckii subsp. bulgaricus* is a lactic acid bacterium. Preferably the milk-based composition is fermented by *S. thermophilus* in the absence of other lactic acid bacteria.

As is known in the art the first step in a process for the fermentation of a milk-based composition is typically a pasteurization step. This step is performed is performed in order to remove any acidifying bacteria that may be present in the fermented milk-based composition itself before fermentation.

Preferably *S. thermophilus* is provided in a concentration of at least 10⁴ CFU per gram of milk-based composition, more preferably in a concentration of at least 10⁵ CFU per gram of milk-based composition, even more preferably in a concentration of at least 10⁶ CFU per gram of milk-based composition, most preferably in a concentration of at least 10⁷ CFU per gram of milk-based composition. The term "milk-based composition" herein refers to the milk-based composition that is to be fermented.

As described above, the invention concerns the use of formate for increasing the thickness of a fermented milk-based composition, wherein the formate is provided to the milk-based composition before and/or during fermentation, preferably before fermentation. An additional advantage of the use of formate is that the fermentation time is decreased. An alternative wording for decreasing the fermentation time is increasing the acidification rate.

The present invention thus also relates to the use of formate for increasing the thickness of a fermented milk-based composition and for decreasing the fermentation time of a fermented milk-based composition, wherein the fermented milk-based composition is fermented by *S. thermophilus* and the formate is provided to the milk-based composition before and/or during fermentation, preferably before fermentation.

Similarly, the invention relates to the use of formate in a starter culture for increasing the thickness of a fermented milk-based composition and for decreasing the fermentation time of a fermented milk-based composition, wherein the starter culture comprises *S. thermophilus.*

The fermentation time is defined as the time period between inoculation of a milk-based composition and the moment the fermented milk-based composition has reached a pH of 4.6. The fermentation time of a fermented milk-based composition is defined as the time period between inoculation of the milk-based composition to be fermented and the moment the fermented milk-based composition has reached a pH of 4.6.

Preferably in the use of formate for decreasing the fermentation time the fermentation time is decreased as compared to a reference fermented milk-based composition fermented by *S. thermophilus* wherein no formate is provided to the reference milk-based composition before and/or during fermentation.

A faster acidification rate inevitably leads to a decrease of fermentation time. The acidification rate is the rate with which the pH drops during fermentation. Preferably in the use of formate for increasing the acidification rate the acidification rate is increased as compared to a reference fermented milk-based composition fermented by S. *thermophilus* wherein no formate is provided to the reference milk-based composition before and/or during fermentation.

### Examples

### Example 1 - Yoghurt fermentation by S. thermophilus

To obtain a milk-based composition full fat cow's milk was skimmed to a fat level of 0.1 gram per 100 ml and subsequently standardized to a protein level of 4.2 gram per 100 ml by adding skimmed milk powder. Subsequently the milk-based composition was sterilized for 15 minutes at 121°C followed by a pasteurization treatment for 5 minutes at 92°C. The milk-based composition was cooled to 7°C and 4 fermentation vessels were filled with 3 kg of the milk-based composition. For the formate comprising variant 0.024 gram per liter sodium formate was provided through adding 0.15 gram of a stock solution (comprising 40 gram sodium formate per 100 ml) to 3 kg of the milk-based composition. Subsequently the milk-based compositions were inoculated and brought to a temperature of 42°C.

The milk-based compositions in the fermentation vessels were inoculated to a level of 1·10⁶ CFU/gram with deep-frozen pellets of a single strain *S. thermophilus* culture C35. C35 is commercially available from CSK Food Enrichment (the Netherlands).

After reaching the pH of 4.60 the fermented milk-based composition was stirred manually 10 times and put through a sieve. Subsequently the fermented milk-based composition was filled in cups of 175 ml, cooled for 45 minutes by placing the cups in a refrigerator having a temperature of 15°C, and finally the cups were stored in a fridge at 7°C.

The different variants were analyzed for viscosity 3 days after first storage and were analyzed for sensory attributes by an expert panel at 7 and 21 days after first storage. Viscosity was measured at a shear rate of 100 s⁻¹ at 20°C in a rheometer with a cone-plate geometry (Anton Paar Physica MCR 301). The time to reach a pH of 4.6 during fermentation was determined. All results are listed in table 1. Only the sensory results towards the mouthfeel attribute thickness are listed in this table.

Surprisingly the variant with additional formate demonstrated a higher viscosity and a higher sensory mouthfeel attribute thickness compared to the variant without formate, as illustrated in Table 1 below.

**Table 1 - Experimental variants and results.**

| **Variant** | **Culture** | **Formate present** | **Time to pH 4.6** | **pH after 7 days** | **Viscosity** | **Sensorial evaluation after 7 days** | **Sensorial evaluation after 21 days** |
|---|---|---|---|---|---|---|---|
| | | | *minutes* | | *mPa·s* | | |
| A | C35 | No | 555 | 4.4 | 480 | | |
| B | C35 | Yes | 281 | 4.4 | 668 | Thicker than A | Thicker than A |

### Example 2 - Yoghurt fermentation by a combination of S. thermophilus and L. bulgaricus

The method of example 1 was repeated for cultures Y520 and Y501; all obtained from CSK Food Enrichment BV (the Netherlands). Cultures Y520 and Y501 are mixed cultures comprising both S. *thermophilus and L. bulgaricus.* In table 2 the experimental variants and results are listed.

Surprisingly, both for Y520 and Y501 the variants with additional formate (F and H) demonstrated a higher viscosity and a higher mouthfeel attribute thickness compared to the variants without formate, as illustrated in Table 2 below.

**Table 2 - Experimental variants and results.**

| **Variant** | **Culture** | **Formate present** | **Time to pH 4.6** | **pH after 7 days** | **Viscosity** | **Sensorial evaluation after 7 days** | **Sensorial evaluation after 21 days** |
|---|---|---|---|---|---|---|---|
| | | | *minutes* | | *mPa·s* | | |
| E | Y520 | No | 538 | 4.4 | 606 | | |
| F | Y520 | Yes | 278 | 4.4 | 686 | Thicker than E | Thicker than E |
| G | Y501 | No | 459 | 4.4 | 230 | | |
| H | Y501 | Yes | 350 | 4.4 | 271 | Thicker than G | Thicker than G |

## Claims

1. Use of formate for increasing the thickness of a fermented milk-based composition, wherein the fermented milk-based composition is fermented by S. *thermophilus* and the formate is provided to the milk-based composition before and/or during fermentation, preferably before fermentation, wherein increasing the thickness is selected from the group consisting of increasing the viscosity, increasing the sensory mouthfeel attribute thickness and the combination thereof.

2. Use of formate in a starter culture for increasing the thickness of a fermented milk-based composition, wherein the starter culture comprises *S*. *thermophilus,* wherein increasing the thickness is selected from the group consisting of increasing the viscosity, increasing the sensory mouthfeel attribute thickness and the combination thereof.

3. The use of formate according to claim 2, wherein the starter culture comprises in the range of 2 to 25 wt.% formate, based on total weight of the starter culture, preferably in the range of 3 to 10 wt.% formate, based on total weight of the starter culture.

4. The use of formate according to any one of claims 1 or 3, wherein the thickness, the viscosity and/or the sensory mouthfeel attribute thickness of the fermented milk-based composition is/are increased as compared to a reference fermented milk-based composition fermented by S. *thermophilus* wherein no formate is provided to the reference milk-based composition before and during fermentation.

5. The use of formate according to any one of claims 2 or 3, wherein the thickness, the viscosity and/or the sensory mouthfeel attribute thickness of the fermented milk-based composition is/are increased as compared to a starter culture not comprising formate.

6. The use of formate according to any one of claims 1, 3, or 4, or the use of formate in a starter culture according to any one of embodiments 2, 3 or 5, wherein the fermented milk-based composition is fermented exclusively by S. *thermophilus,* or wherein the starter culture does not comprise other lactic acid bacteria.

7. The use of formate according to any one of claims 1 or 3-5, or the use of formate in a starter culture according to any one of embodiments 2-5, wherein the fermented milk-based composition is fermented by a combination of S. *thermophilus* and *L. bulgaricus,* or wherein the starter culture comprises a combination of *S. thermophilus* and *L*. *bulgaricus.*

8. The use of formate according to any one of the preceding claims, wherein the fermented milk-based composition comprises at least 50 wt.% of milk-based components, based on dry weight of the milk-based components and based on dry weight of the milk-based composition.

9. The use of formate according to any of the preceding claims, wherein the fermented milk-based composition comprises 2 to 10 wt.% protein, based on total fermented milk-based composition, preferably wherein the protein is milk protein.

10. The use of formate according to any of the preceding claims, wherein formate is selected from the group consisting of formic acid, sodium formate, potassium formate, ammonium formate, calcium formate, magnesium formate, ferric formate, zinc formate, copper formate, ethyl formate, methyl formate and any combination thereof, preferably formate is selected from the group consisting of formic acid, sodium formate, potassium formate, ammonium formate, calcium formate and any combination thereof, most preferably formate is selected from the group consisting of sodium formate, formic acid and a combination thereof.

11. The use of formate according to any of the preceding claims, wherein the fermented milk-based composition is selected from the group consisting of yoghurt and fresh cheese, preferably the fermented milk-based composition is yoghurt.

12. The use of formate according to any of the preceding claims, wherein formate is provided to the milk-based composition in an amount of 2 to 100 microgram formate per gram of milk-based composition, preferably wherein formate is provided to the milk-based composition in an amount of 3 to 50 microgram formate per gram of milk-based composition.

13. The use of formate according to any of the preceding claims, wherein S. *thermophilus* is provided to the milk-based composition at the same moment as the formate is provided.

14. The use of formate according to any of the preceding claims, wherein the milk is selected from the group consisting of cow's milk, goats milk, sheep milk, almond milk, oat milk, and soy milk, preferably wherein the milk is cow's milk.

## Patentansprüche

1. Verwendung von Formiat zur Erhöhung der Dicke einer vergorenen Zusammensetzung auf Milchbasis, wobei die vergorene Zusammensetzung auf Milchbasis durch *S. thermophilus* vergoren und das Formiat der vergorenen Zusammensetzung auf Milchbasis vor und/oder während der Fermentation, bevorzugt vor der Fermentation, zugeführt wird, wobei Erhöhen der Dicke aus der Gruppe bestehend aus Erhöhen der Viskosität, Erhöhen der im Mund als sensorische Eigenschaft wahrgenommenen Dicke und der Kombination davon ausgewählt ist.

2. Verwendung von Formiat in einer Starterkultur zur Erhöhung der Dicke einer vergorenen Zusammensetzung auf Milchbasis, wobei die Starterkultur *S. thermophilus* umfasst, wobei Erhöhen der Dicke aus der Gruppe bestehend aus Erhöhen der Viskosität, Erhöhen der im Mund als sensorische Eigenschaft wahrgenommenen Dicke und der Kombination davon ausgewählt ist.

3. Verwendung von Formiat nach Anspruch 2, wobei die Starterkultur Formiat im Bereich von 2 bis 25 Gew.-%, bezogen auf das Gesamtgewicht der Starterkultur, bevorzugt im Bereich von 3 bis 10 Gew.-%, bezogen auf das Gesamtgewicht der Starterkultur, umfasst.

4. Verwendung von Formiat nach einem der Ansprüche 1 oder 3, wobei die Dicke, die Viskosität und/oder die im Mund als sensorische Eigenschaft wahrgenommene Dicke der vergorenen Zusammensetzung auf Milchbasis verglichen mit einer vergorenen Referenz-Zusammensetzung auf Milchbasis, die durch *S. thermophilus* vergoren wird, erhöht ist/sind, wobei der vergorenen Referenz-Zusammensetzung auf Milchbasis vor und während der Fermentation kein Formiat zugeführt wird.

5. Verwendung von Formiat nach einem der Ansprüche 2 oder 3, wobei die Dicke, die Viskosität und/oder die im Mund als sensorische Eigenschaft wahrgenommene Dicke der vergorenen Zusammensetzung auf Milchbasis verglichen mit einer Starterkultur, die kein Formiat umfasst, erhöht ist/sind.

6. Verwendung von Formiat nach einem der Ansprüche 1, 3 oder 4 oder Verwendung von Formiat in einer Starterkultur nach einer der Ausführungsformen 2, 3 oder 5, wobei die vergorene Zusammensetzung auf Milchbasis ausschließlich durch *S. thermophilus* vergoren wird oder wobei die Starterkultur keine anderen Milchsäurebakterien umfasst.

7. Verwendung von Formiat nach einem der Ansprüche 1 oder 3-5 oder Verwendung von Formiat in einer Starterkultur nach einer der Ausführungsformen 2-5, wobei die vergorene Zusammensetzung auf Milchbasis durch eine Kombination von *S. thermophilus* und *L. bulgaricus* vergoren wird oder wobei die Starterkultur eine Kombination von *S. thermophilus* und *L. bulgaricus* umfasst.

8. Verwendung von Formiat nach einem der vorhergehenden Ansprüche, wobei die vergorene Zusammensetzung auf Milchbasis wenigstens 50 Gew.-% Komponenten auf Milchbasis, bezogen auf das Trockengewicht der Komponenten auf Milchbasis und bezogen auf das Trockengewicht der Zusammensetzung auf Milchbasis, umfasst.

9. Verwendung von Formiat nach einem der vorhergehenden Ansprüche, wobei die vergorene Zusammensetzung auf Milchbasis 2 bis 10 Gew.-% Protein, bezogen auf die gesamte vergorene Zusammensetzung auf Milchbasis, umfasst, vorzugsweise wobei es sich bei dem Protein um Milchprotein handelt.

10. Verwendung von Formiat nach einem der vorhergehenden Ansprüche, wobei Formiat aus der Gruppe bestehend aus Ameisensäure, Natriumformiat, Kaliumformiat, Ammoniumformiat, Calciumformiat, Magnesiumformiat, Eisen(III)formiat, Zinkformiat, Kupferformiat, Ameisensäureethylester, Ameisensäuremethylester und irgendeiner Kombination davon, bevorzugt aus der Gruppe bestehend aus Ameisensäure, Natriumformiat, Kaliumformiat, Ammoniumformiat, Calciumformiat und irgendeiner Kombination davon, am meisten bevorzugt aus der Gruppe bestehend aus Natriumformiat, Ameisensäure und einer Kombination davon ausgewählt ist.

11. Verwendung von Formiat nach einem der vorhergehenden Ansprüche, wobei die vergorene Zusammensetzung auf Milchbasis aus der Gruppe bestehend aus Joghurt und Frischkäse ausgewählt ist, wobei es sich bevorzugt bei der vergorenen Zusammensetzung auf Milchbasis um Joghurt handelt.

12. Verwendung von Formiat nach einem der vorhergehenden Ansprüche, wobei Formiat der Zusammensetzung auf Milchbasis in einer Menge von 2 bis 100 Mikrogramm Formiat pro Gramm Zusammensetzung auf Milchbasis zugeführt wird, vorzugsweise wobei Formiat der Zusammensetzung auf Milchbasis in einer Menge von 3 bis 50 Mikrogramm Formiat pro Gramm Zusammensetzung auf Milchbasis zugeführt wird.

13. Verwendung von Formiat nach einem der vorhergehenden Ansprüche, wobei *S. thermophilus* der Zusammensetzung auf Milchbasis gleichzeitig mit dem Formiat zugeführt wird.

14. Verwendung von Formiat nach einem der vorhergehenden Ansprüche, wobei die Milch aus der Gruppe bestehend aus Kuhmilch, Ziegenmilch, Schafsmilch, Mandelmilch, Hafermilch und Sojamilch ausgewählt ist, vorzugsweise wobei es sich bei der Milch um Kuhmilch handelt.

## Revendications

1. Utilisation de formiate pour augmenter l'épaisseur d'une composition à base de lait fermenté, la composition à base de lait fermenté étant fermentée par *S. thermophilus* et le formiate étant fourni à la composition à base de lait avant et/ou pendant la fermentation, préférablement avant la fermentation, l'augmentation de l'épaisseur étant choisie dans le groupe constitué par une augmentation de la viscosité, une augmentation de l'épaisseur de sensation bucco-tactile sensorielle et la combinaison correspondante.

2. Utilisation de formiate dans une culture starter pour augmenter l'épaisseur d'une composition à base de lait fermenté, la culture starter comprenant S. *thermophilus,* l'augmentation de l'épaisseur étant choisie dans le groupe constitué par une augmentation de la viscosité, une augmentation de l'épaisseur de sensation bucco-tactile sensorielle et la combinaison correspondante.

3. Utilisation de formiate selon la revendication 2, la culture starter comprenant dans la plage de 2 à 25 % en poids de formiate, sur la base du poids total de la culture starter, préférablement dans la plage de 3 à 10 % en poids de formiate, sur la base du poids total de la culture starter.

4. Utilisation de formiate selon l'une quelconque des revendications 1 ou 3, l'épaisseur, la viscosité et/ou l'épaisseur de sensation bucco-tactile sensorielle de la composition à base de lait fermenté étant augmentée(s) par comparaison avec une composition à base de lait fermenté de référence fermentée par *S. thermophilus* dans laquelle du formiate n'est pas fourni à la composition à base de lait de référence avant et pendant la fermentation.

5. Utilisation de formiate selon l'une quelconque des revendications 2 ou 3, l'épaisseur, la viscosité et/ou l'épaisseur de sensation bucco-tactile sensorielle de la composition à base de lait fermenté étant augmentée(s) par comparaison avec une culture starter ne comprenant pas de formiate.

6. Utilisation de formiate selon l'une quelconque des revendications 1, 3 ou 4, ou utilisation de formiate dans une culture starter selon l'un quelconque des modes de réalisation 2, 3 ou 5, la composition à base de lait fermenté étant fermentée exclusivement par *S. thermophilus,* ou la culture starter ne comprenant pas d'autres bactéries d'acide lactique.

7. Utilisation de formiate selon l'une quelconque des revendications 1 ou 3-5, ou utilisation de formiate dans une culture starter selon l'un quelconque des modes de réalisation 2-5, la composition à base de lait fermenté étant fermentée par une combinaison de *S. thermophilus* et de *L. bulgaricus,* ou la culture starter comprenant une combinaison de *S. thermophilus* et de *L. bulgaricus.*

8. Utilisation de formiate selon l'une quelconque des revendications précédentes, la composition à base de lait fermenté comprenant au moins 50 % en poids de composants à base de lait, sur la base du poids sec des composants à base de lait et basé sur le poids sec de la composition à base de lait.

9. Utilisation de formiate selon l'une quelconque des revendications précédentes, la composition à base de lait fermenté comprenant 2 à 10 % en poids de protéine, sur la base de la composition à base de lait fermenté totale, préférablement la protéine étant une protéine de lait.

10. Utilisation de formiate selon l'une quelconque des revendications précédentes, le formiate étant choisi dans le groupe constitué par l'acide formique, le formiate de sodium, le formiate de potassium, le formiate d'ammonium, le formiate de calcium, le formiate de magnésium, le formiate ferrique, le formiate de zinc, le formiate de cuivre, le formiate d'éthyle, le formiate de méthyle et une quelconque combinaison correspondante, préférablement le formiate étant choisi dans le groupe constitué par l'acide formique, le formiate de sodium, le formiate de potassium, le formiate d'ammonium, le formiate de calcium et une quelconque combinaison correspondante, le plus préférablement le formiate étant choisi dans le groupe constitué par le formiate de sodium, l'acide formique et une combinaison correspondante.

11. Utilisation de formiate selon l'une quelconque des revendications précédentes, la composition à base de lait fermenté étant choisie dans le groupe constitué par un yogourt et du fromage frais, préférablement la composition à base de lait fermenté étant un yogourt.

12. Utilisation de formiate selon l'une quelconque des revendications précédentes, le formiate étant fourni à la composition à base de lait en une quantité de 2 à 100 microgrammes de formiate par gramme de composition à base de lait, préférablement le formiate étant fourni à la composition à base de lait en une quantité de 3 à 50 microgrammes de formiate par gramme de composition à base de lait.

13. Utilisation de formiate selon l'une quelconque des revendications précédentes, *S. thermophilus* étant fourni à la composition à base de lait au même moment que le formiate est fourni.

14. Utilisation de formiate selon l'une quelconque des revendications précédentes, le lait étant choisi dans le groupe constitué par lait de vaches, le lait de chèvres, le lait de moutons, le lait d'amande, le lait d'avoine et le lait de soja, préférablement le lait étant le lait de vaches.
